(19) Europäisches Patentamt
European Patent Office
Office européen des brevets

(11) Veröffentlichungsnummer: **0 262 533 B1**

(12) **EUROPÄISCHE PATENTSCHRIFT**

(45) Veröffentlichungstag der Patentschrift: **20.01.93**

(51) Int. Cl.[5]: **C07C 45/65**, C07C 49/203, B01J 29/04

(21) Anmeldenummer: **87113731.1**

(22) Anmeldetag: **19.09.87**

# (54) Verfahren zur Herstellung von alpha,beta-ungesättigten Ketonen.

(30) Priorität: **25.09.86 DE 3632530**

(43) Veröffentlichungstag der Anmeldung:
**06.04.88 Patentblatt 88/14**

(45) Bekanntmachung des Hinweises auf die Patenterteilung:
**20.01.93 Patentblatt 93/03**

(84) Benannte Vertragsstaaten:
**BE CH DE FR GB LI NL**

(56) Entgegenhaltungen:
**GB-A- 2 008 573**
**GB-A- 2 010 252**

**METHODEN DER ORGANISCHEN CHEMIE, Band VII/2c, 4. Auflage, 1977, Seiten 2115-2116, Georg Thieme Verlag, Stuttgart ; HOUBEN-WEYL**

**BEILSTEINS HANDBUCH DER ORGANISCHEN CHEMIE, Band 1, Ergänzungswerk III, 3. Teil, 1959, Springer Verlag, Seite 2987, Berlin, Seite 2987, Zeilen 37-40**

**PATENT ABSTRACTS OF JAPAN, Band 10, Nr. 144 (C-349)(2201), 27. Mai 1986 & JP-A-615038**

(73) Patentinhaber: **BASF Aktiengesellschaft**
**Carl-Bosch-Strasse 38**
**W-6700 Ludwigshafen(DE)**

(72) Erfinder: **Hoelderich, Wolfgang, Dr.**
**Mannheimer Strasse 18 c**
**W-6710 Frankenthal(DE)**
Erfinder: **Schneider, Kurt, Dr.**
**Auf dem Koeppel**
**W-6702 Bad Duerkheim(DE)**
Erfinder: **Hupfer, Leopold, Dr.**
**Waltershoehe 3**
**W-6701 Friedelsheim(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

**Beschreibung**

Bifunktionelle Verbindungen sind wertvolle Bausteine für organische Synthesen. Zu dieser Verbindungsklasse gehören alpha,beta-ungesättigte Ketone. Diese Ketone werden u.a. zur Synthese von Verbindungen mit physiologischer Aktivität verwendet.

Ein weiteres Anwendungsgebiet für die alpha, beta-ungesättigten Ketone, insbesondere für Methylisopropenylketon liegt in der Chemie von temperaturstabilen Polymeren und Copolymeren. Methylisopropenylketon wird als Monomeres für Poly(isopropenylmethylketon) oder als Copolymerisat mit Polybutadien bzw. PVC verwendet. Weiterhin finden derartige Ketone in der Herstellung von Epoxidharzen Verwendung.

Es ist bekannt, alpha,beta-ungesättigte Ketone durch Aldolkondensation von aliphatischen Ketonen und Formaldehyd in Gegenwart von Oxalsäure bzw. in Gegenwart von Kationenaustauschern und $H_2O$ unter erhöhtem Druck (GB-PS 993 389 oder FR-PS 1 383 548) oder durch Reaktion von Enolacetaten mit Ketonen in Gegenwart von Lewis-Säuren wie $BF_3$, $TiCl_4$ oder durch Oxidation verzweigter Olefine in 50 %iger Essigsäure und in Gegenwart von $PdCl_2$ als Katalysator (BE-PS 658 801) oder durch Kondensation von gesättigten Carbonylverbindungen in Gegenwart von $BF_3$ herzustellen.

Daneben gibt es ein mehrstufiges Verfahren, bei dem man alpha,beta-ungesättigte Ketone durch Kondensation von Ketonen in Gegenwart von Tosylmethylisocyanid, anschließende Alkylierung und Hydrolyse erhält. Andere aufwendige, kostenintensive Herstellverfahren sind z.B. die Ozonolyse von 2,3-Dimethylbutadienen oder die anodische Oxidation von beta-Ketocarboxylaten oder die Photoisomerisation von 1,2-Diacylcyclobutanen. Die Gasphasenoxidation von Olefinen an Metalloxid-Phosphoroxid-Katalysatoren führt unselektiv zu einem Gemisch verschiedener Verbindungen, unter denen sich auch alpha, beta-ungesättigte Ketone befinden.

Die bekannten Verfahren weisen verschiedene Nachteile auf, sei es, daß man von schwer zugänglichen Ausgangsverbindungen ausgehen, daß man toxische und korrosive Homogenkatalysatoren verwenden, oder daß man eine energieaufwendige oder mehrstufige Reaktionsführung wie Ozonolyse bzw. Photoisomerisation in Kauf nehmen muß.

Daraus ergab sich die Aufgabe, alpha,beta-ungesättigte Ketone der Formel (I) durch eine einfache Reaktion aus besser zugänglichen Ausgangsverbindungen zu synthetisieren.

Es wurde gefunden, daß man die oben beschriebenen Nachteile vermeidet und alpha,beta-ungesättigte Ketone der Formel (I)

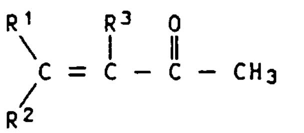

(I),

in der $R^1$, $R^2$, $R^3$ Wasserstoff, Alkyl- und/oder Alkenyl- mit 1 bis 12 Kohlenstoffatomen, Cycloalkyl-, Aryl-, Aralkyl- bzw. Alkylarylreste, die ihrerseits substituiert sein können, bedeuten bzw. $R^1$ und $R^3$ oder $R^2$ und $R^3$ zusammen mit den C-Atomen, an die sie gebunden sind, ein Cycloalkan oder Cycloalken bilden können, in einfacher Weise erhält, wenn man Ketone der Formel (II)

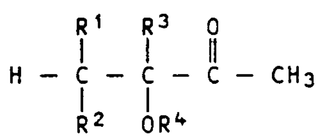

(II),

in der $R^1$ , $R^2$, $R^3$ obige Bedeutung haben und $R^4$ gleich Wasserstoff, Alkyl- oder einem Carboxylrest bedeutet, in Gegenwart von sauren Heterogenkatalysatoren umsetzt.

Ketone der Formel (II) die erfindungsgemäß als Ausgangsstoffe bevorzugt umgesetzt werden können, sind z.B. 3-Methyl-3-hydroxy-butan-2-on, 3-Methyl-3-hydroxy-pentan-2-on, 3-Pentamethylen-3-hydroxypropan-2-on sowie auch 3-Methyl-3-hydroxy-heptan-2-on und 3-Phenyl-3-hydroxy-3-methylpropan-2-on. Die Ausgangsstoffe kann man z.B. nach der in DE-PS 11 29 941 beschriebenen Verfahrensweise aus tert.-Acetylalkoholen herstellen.

Als Heterogenkatalysatoren im Sinne der Erfindung sind im allgemeinen geeignet Zeolithe des Pentasiltyps wie Aluminiumsilikatzeolithe, Borosilikatzeolithe. Eisensilikatzeolithe, und Zeolithe des Faujasit-Typs.

Die Zeolithe können z.B. mit Alkalimetallen, Übergangsmetallen und mit seltenen Erdmetallen dotiert

werden.

Man kann als Katalysatoren aber auch Phosphate der Elemente B, Al, Zr, Fe. Sr oder deren Gemische verwenden. Auch hydrothermal hergestellte Phosphate sind z.B. als Katalysatoren geeignet z.B. hydrothermal hergestellte Aluminiumphosphate, Siliciumaluminiumphosphate oder Siliciumeisenaluminiumphosphate. Daneben kann man als Katalysatoren Phosphorsäure auf Trägermaterialien verwenden.

Als Heterogenkatalysatoren für das erfindungsgemäße Verfahren verwendet man die Zeolithe zweckmäßig in der aciden Form. Zeolithe sind kristalline Aluminiumsilikate, die eine hochgeordnete Struktur mit einem starren dreidimensionalen Netzwerk von $SiO_4$- und $AlO_4$-Tetraedern besitzen, die durch gemeinsame Sauerstoffatome verbunden sind. Das Verhältnis der Si-und Al-Atome zu Sauerstoff beträgt 1 : 2. Die Elektrovalenz der Aluminium enthaltenden Tetraeder ist durch Einschluß von Kationen in den Kristall, z.B. eines Alkali- oder Wasserstoffions ausgeglichen. Ein Kationenaustausch ist möglich. Die Räume zwischen den Tetraedern sind vor der Dehydration durch Trocknen bzw. Calcinieren von Wassermolekülen besetzt.

In den Zeolithen können anstelle von Aluminium auch andere Elemente wie B, Ga, Fe, Cr, V, As, Sb, Bi oder Be oder deren Gemische in das Gitter eingebaut werden, oder das Silicium kann durch ein vierwertiges Element wie Ge, Ti, Zr, Hf ersetzt werden.

Entsprechend ihrer Struktur werden Zeolithe in verschiedene Gruppen unterteilt. So bilden bei der Mordenit-Gruppe Ketten oder bei der Chabasit-Gruppe Schichten aus Tetraedern die Zeolith-Struktur, während sich bei der Faujasit-Gruppe die Tetraeder zu Polyedern ordnen, z.B. in Form eines Kubooktaeders, der aus Vierringen bzw. Sechsringen aufgebaut ist. Je nach Verknüpfung der Kubooktaeder, wodurch unterschiedlich große Hohlräume und Poren entstehen, unterscheidet man in Zeolithe vom Typ A, L, X oder Y.

Für das erfindungsgemäße Verfahren in Betracht kommende Katalysatoren sind Zeolithe aus der Mordernit-Gruppe oder engporige Zeolithe vom Erionit-bzw. Chabasit-Typ oder Zeolithe vom Faujasit-Typ, z.B. Y-, X- oder L-Zeolithe.

In dieser Gruppe von Zeolithen gehören auch die sogenannten "ultrastabilen" Zeolithe des Faujasittyps, d.h. dealuminierte Zeolithe. Verfahren zur Herstellung solcher Zeolithe sind mannigfach beschrieben.

Besonders vorteilhaft sind Zeolithe vom Pentasiltyp. Diese haben als Grundbaustein einen aus $SiO_4$-Tetraedern aufgebauten Fünfring gemeinsam. Sie sind durch eine hohes $SiO_2/Al_2O_3$-Verhältnis gekennzeichnet sowie durch Porengrößen, die zwischen denen der Zeolithe vom Typ A und denen vom Typ X oder Y liegen.

Diese Zeolithe können unterschiedliche chemische Zusammensetzung aufweisen. Es handelt sich hierbei um Alumino-, Boro-, Eisen-, Beryllium-, Gallium-, Chrom-, Arsen-, Antimon- und Wismutsilikatzeolithe oder deren Gemische sowie Alumino-, Boro, Gallium- und Eisengermanatzeolithe oder deren Gemische. Insbesondere eignen sich die Alumino-, Boro- und Eisensilikatzeolithe des Pentasiltyps für das erfindungsgemäße Verfahren. Der Aluminosilkikatzeolith wird z.B. aus einer Aluminiumverbindung, vorzugsweise $Al(OH)_3$ oder $Al_2(SO_4)_3$ und einer Siliciumkomponente, vorzugsweise hochdispersem Siliciumdioxid in wäßriger Aminlösung, insbesondere in Polyaminen wie 1,6-Hexandiamin- oder 1,3-Propandiamin- oder Triethylentetramin-Lösung mit oder insbesondere ohne Alkali- oder Erdalkalizusatz bei 100 bis 220 °C unter autogenem Druck hergestellt. Hierzu gehören auch die isotaktischen Zeolithe nach EP 34727 und EP 46504. Die erhaltenen Aluminosilikatzeolithe enthalten je nach Wahl der Einsatzstoffmengen ein $SiO_2/Al_2O_3$-Verhältnis von 10 bis 40 000. Aluminosilikatzeolithe des Pentasiltyps können auch in etherischem Medium wie Diethylenglykoldimethylether, in alkoholischem Medium wie Methanol bzw. 1,4-Butandiol oder in Wasser synthetisiert werden.

Zu den erfindungsgemäß verwendbaren siliciumreichen Zeolithen ($SiO_2/Al_2O_3 \geqq 10$) gehören auch die verschiedenen ZSM-Typen, Ferrierit, Nu-1 und Silicalit ®.

Borosilikatzeolithe kann man z.B. bei 90 bis 200 °C unter autogenem Druck synthetisieren, indem man eine Borverbindung, z.B. $H_3BO_3$, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetramin-Lösung mit und insbesondere ohne Alkali- oder Erdalkalizusatz zur Reaktion bringt. Hierzu gehören auch die isotaktischen Zeolithe nach EP 34 727 und EP 46 504. Solche Borosilikatzeolithe können ebenfalls hergestellt werden, wenn man die Reaktion statt in wäßriger Aminlösung in etherischer Lösung, z.B. Diethylenglykoldimethylether oder in alkoholischer Lösung, z.B. 1,6-Hexandiol durchführt.

Den Eisensilikatzeolith erhält man z.B. aus einer Eisenverbindung, vorzugsweise $Fe_2(SO_4)_3$ und einer Siliciumverbindung, vorzugsweise hochdispersem Siliciumdioxid in wäßriger Aminlösung, insbesondere 1,6-Hexandiamin, mit oder ohne Alkali- oder Erdalkalizusatz bei 100 bis 200 °C unter autogenem Druck.

Die so hergestellten Alumino-, Boro- und Eisensilikatzeolithe können nach ihrer Isolierung, Trocknung bei 100 bis 160 °C, vorzugsweise 110 °C und Calcinierung bei 450 bis 550 °C, vorzugsweise 500 °C, mit einem Bindemittel im Verhältnis 90 : 10 bis 40 : 60 Gew.-% zu Strängen oder Tabeletten verformt werden.

Als Bindemittel eignen sich diverse Aluminiumoxide, bevorzugt Boehmit, amorphe Aluminosilikate mit einem $SiO_2/Al_2O_3$-Verhältnis von 25 : 75 bis 90 : 5, bevorzugt 75 : 25, Siliciumdioxid, bevorzugt hochdisperses $SiO_2$, Gemische aus hochdispersem $SiO_2$ und hochdispersem $Al_2O_3$, $TiO_2$, $ZrO_2$ sowie Ton. Nach der Verformung werden die Extrudate oder Preßlinge bei 110 ° C/16 h getrocknet und bei 500 ° C/16 h calciniert.

Man erhält auch vorteilhafte Katalysatoren, wenn der isolierte Alumino-bzw., Borosilikatzeolith direkt nach der Trocknung verformt wird und erst nach der Verformung einer Calcinierung unterworfen wird. Die hergestellten Alumino- und Borosilikatzeolithe können in reiner Form, ohne Binder, als Stränge oder Tabletten eingesetzt werden, wobei als Verstrangungs- oder Peptisierungshilfsmittel, z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden.

Liegt der Zeolith aufgrund der Art seiner Herstellung nicht in der katalytisch aktiven, aciden H-Form vor, sondern z.B. in der Na-Form, dann kann diese durch Ionenaustausch, z.B. mit Ammoniumionen und anschließende Calcinierung oder durch Behandlung mit Säuren vollkommen oder partiell in die gewünschte H-Form überführt werden.

Wenn bei der erfindungsgemäßen Verwendung der zeolithischen Katalysatoren eventuell eine durch Koksabscheidung bedingte Desaktivierung eintritt, empfiehlt es sich, die Zeolithe durch Abbrennen der Koksablagerung mit Luft oder mit einem Luft/$N_2$-Gemisch bei 400 bis 550 ° C, bevorzugt 500 ° C, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Um eine möglichst hohe Selektivität, hohen Umsätzen sowie lange Standzeiten zu erreichen, ist es vorteilhaft, die Zeolithe zu modifizieren. Eine geeignete Modifizierung der Katalysatoren besteht z.B. darin, daß man den unverformten oder verformten Zeolithen mit Metallsalzen durch einen Ionenaustausch oder durch Imprägnierung dotiert. Als Metalle werden Alkalimetalle wie Li, Cs, K, Erdalkalimetall wie Mg, Ca, Sr, Metalle der 3., 4. und 5. Hauptgruppe wie Al, Ga, Ge, Sn, Pb, Bi, Übergangsmetalle der 4. - 8. Nebengruppe wie Ti, Zr, V, Nb, Cr, Mo, W, Mn, Re, Fe, Ru, Os, Co, Rh, Sr, Ni, Pd, Pt, Übergangsmetalle der 1. und 2. Nebengruppe wie Cu, Ag, Zn, Seltene Erdmetalle wie La, Ce, Pr, Nd, Er, Yb und U eingesetzt.

Zweckmäßigerweise führt man die Dotierung so durch, daß man den verformten Zeolithen in einem Steigrohr vorlegt und bei 20 bis 100 ° C eine wäßrige oder ammonialkalische Lösung eines Halogenids oder eines Nitrats der voranbeschriebenen Metalle überleitet. Ein derartiger Ionenaustausch kann an der Wasserstoff-, Ammonium- und Alkaliform des Zeolithen vorgenommen werden. Eine weitere Möglichkeit der Metallaufbringung auf den Zeolithen ist gegeben, indem man das zeolithische Material, z.B. mit einem Halogenid, einem Nitrat oder einem Oxid der voranbeschriebenen Metalle in wäßriger, alkoholischer oder ammonialkalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht z.B. darin, daß man $Cu(NO_3)_2 \times 3\ H_2O$ oder $Ni(NO_3)_2 \times 6\ H_2O$ oder $Ce(NO_3)_3 \times 6\ H_2O$ oder $La(NO_3)_2 \times 6\ H_2O$ oder $Cs_2CO_3$ in Wasser löst und mit dieser Lösung den verformten oder unverformten Zeolith eine gewisse Zeit, z.B. 30 Minuten, tränkt. Die eventuell überstehende Lösung wird im Rotationsverdampfer von Wasser befreit. Danach wird der getränkte Zeolith bei etwa 150 ° C getrocknet und bei etwa 550 ° C calciniert. Dieser Tränkvorgang kann mehrmals hintereinander vorgenommen werden, um den gewünschten Metallgehalt einzustellen.

Es ist auch möglich, eine wäßrige $Ni(CO_3)_2$-Lösung oder ammoniakalische $Pd(NO_3)_2$-Lösung herzustellen und darin den reinen pulverförmigen Zeolithen bei 40 bis 100 ° C unter Rühren etwa 24 h aufzuschlämmen. Nach Abfiltrieren, Trocknen bei etwa 150 ° C und Calcinierung bei etwa 500 ° C kann das so gewonnene zeolithische Material mit oder ohne Bindemittel zu Strängen, Pellets oder Wirbelgut weiterverarbeitet werden.

Ein Ionenaustausch des in der H-Form oder Ammonium-Form oder Alkali-Form vorliegenden Zeolithen kann so vorgenommen werden, daß man den Zeolithen in Strängen oder Pellets in einer Kolonne vorlegt und darüber z.B. eine wäßrige $Ni(NO_3)_2$-Lösung oder ammoniakalische $Pd(NO_3)_2$-Lösung bei leicht erhöhter Temperatur zwischen 30 und 80 ° C im Kreislauf 15 bis 20 h leitet. Danach wird mit Wasser ausgewaschen, bei etwa 150 ° C getrocknet und bei etwa 550 ° C calciniert. Bei manchen metalldotierten Zeolithen, z.B. Pd-, Cu-, Ni-dotierten Zeolithen ist eine Nachbehandlung mit Wasserstoff vorteilhaft.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft. Dabei geht man vorteilhaft, z.B. so vor, daß man Zeolithe in Pulverform mit 1 n Phosphorsäure 1 Stunde bei 80 ° C behandelt. Nach der Behandlung wird mit Wasser gewaschen, bei 110 ° C/16 Stunden getrocknet und bei 500 ° C/20 Stunden calciniert. Nach einer anderen Arbeitsweise behandelt man Zeolithe vor oder nach ihrer Verformung mit Bindemittel, z.B. 1 bis 3 Stunden bei

Temperaturen von 60 bis 80 °C mit einer 3 bis 25 Gew.-%igen, insbesondere 12 bis 20 Gew.-%igen wäßrigen Salzsäure. Anschließend wird der so behandelte Zeolith mit Wasser gewaschen, getrocknet und bei 400 °C bis 500 °C calciniert.

Eine besondere Ausführungsform für die Säurebehandlung besteht darin, daß man das zeolithische Material vor seiner Vorformung bei erhöhter Temperatur mit Flußsäure, die im allgemeinen als 0,001 n bis 2 n, vorzugsweise 0,05 n bis 0,5 n Flußsäure eingesetzt wird, behandelt, beispielsweise durch Erhitzen unter Rückfluß über einen Zeitraum von im allgemeinen 0,5 bis 5, vorzugsweise 1 bis 3 Stunden. Nach Isolierung, z.B. durch Abfiltrieren und Auswaschen des zeolithischen Materials, wird dieses zweckmäßig, z.B. bei Temperaturen von 100 bis 160 °C, getrocknet und bei Temperaturen von im allgemeinen 450 °C bis 600 °C calciniert. Gemäß einer weiteren bevorzugten Ausführungsform für die Säurebehandlung, wird das zeolithische Material nach einer Verformung mit Bindemittel bei erhöhter Temperatur, zweckmäßig bei Temperaturen von 50 bis 90 °C, vorzugsweise 60 bis 80 °C, über einen Zeitraum von 0,5 bis 5 h, vorzugsweise mit 12 bis 20 Gew.-%iger Salzsäure, behandelt. Zweckmäßig wird das zeolithische Material anschließend ausgewaschen, bei Temperaturen von 100 bis 160 °C getrocknet und bei Temperaturen von 450 bis 600 °C calciniert. Einer HF-Behandlung kann sich auch eine HCl-Behandlung anschließen.

Nach einer anderen Arbeitsweise kann man Zeolithe durch Aufbringen von Phosphorverbindungen, wie Trimethoxiphosphat, Trimethoxiphosphin, primärem, sekundärem oder tertiärem Natriumphosphat modifizieren. Besonders vorteilhaft hat sich die Benandlung mit primärem Natriumphosphat erwiesen. Hierbei werden die Zeolithe in Strang-, Tabletten- oder Wirbelgut-Form mit wäßriger $NaH_2PO_4$-Lösung getränkt, bei 110 °C getrocknet und bei 500 °C calciniert.

Weitere Heterogenkatalysatoren für die Herstellung von alpha,beta-ungesättigten Ketonen sind Phosphate, insbesondere Aluminiumphosphate, Siliciumaluminiumphosphate, Siliciumeisenaluminiumphosphate, Cerphosphat, Zirkonphosphate, Borphosphat, Eisenphosphat oder deren Gemische.

Als Aluminiumphosphat-Heterogenkatalysatoren werden für das erfindungsgemäße Verfahren, insbesondere unter hydrothermalen Bedingungen synthetisierte Aluminiumphosphate eingesetzt. Geeignete Aluminiumphosphate sind z.B. APO-5, APO-9, APO-11, APO-12, APO-14, APO-21 , APO-25, APO-31 und APO-33.

Beispielsweise $AlPO_4$-5 (APO-5) wird synthetisiert, indem man Orthophosphorsäure mit Pseudoboehmit (Catapal SB ®) in Wasser homogen mischt; zu dieser Mischung Tetrapropylammoniumhydroxid gibt und danach bei etwa 150 °C 20 bis 60 h unter autogenem Druck in einem Autoklaven umsetzt. Das abfiltrierte $AlPO_4$ wird getrocknet bei 100 bis 160 °C und calciniert bei 450 bis 550 °C.

$AlPO_4$-9 (APO-9) wird ebenfalls aus Orthophosphorsäure und Pseudoboehmit aber in wäßriger DABCO-Lösung (1,4-Diazabicyclo-(2,2,2)octan) bei ca. 200 °C unter autogenem Druck während 200 bis 400 h synthetisiert. Verwendet man anstelle DABCO-Lösung Ethylendiamin, so gelangt man zu APO-12.

Die Synthese des $AlPO_4$-21 (APO-21) erfolgt aus Orthophosphorsäure und Pseudoboehmit in wäßriger Pyrrolidon-Lösung bei 150 bis 200 °C unter autogenem Druck während 50 bis 200 h.

Für das erfindungsgemäße Verfahren kann man auch bekannte Siliciumaluminiumphosphate wie SAPO-5, SAPO-11, SAPO-31 und SAPO-34 einsetzen. Diese Verbindungen werden hergestellt durch Kristallisation aus wäßriger Mischung bei 100 bis 250 °C und autogenem Druck während 2 h bis 2 Wochen, wobei die Reaktionsmischung aus einer Silicium-, Aluminium- und Phosphorkomponente in wäßrigen aminoorganischen Lösungen umgesetzt wird.

SAPO-5 beispielsweise wird durch Mischen von $SiO_2$ suspendiert in wäßriger Tetrapropylammoniumhydroxid-Lösung mit einer wäßrigen Suspension aus Pseudoboehmit und Orthophosphorsäure und anschließende Umsetzung bei 150 bis 200 °C, während 20 bis 200 h unter autogenem Druck in einem Rührautoklaven erhalten. Die Trocknung des abfiltrierten Pulvers erfolgt bei 110 bis 160 °C und die Calcination bei 450 bis 550 °C.

Als Siliciumaluminiumphosphate sind auch ZYT-5, ZYT-6, ZYT-7, ZYT-9, ZYT-11 und ZYT-12 geeignet.

Als Phosphat-Katalysatoren kann man bei dem Verfahren gefällte Aluminiumphosphate einsetzen. Beispielsweise wird ein derartiges Aluminiumphosphat hergestellt, indem 92 g Diammoniumhydrogenphosphat in 700 ml Wasser gelöst werden. Zu dieser Lösung wird 260 g $Al(NO_3)_3$ x $H_2O$ in 700 ml Wasser über 2 h zugetropft. Dabei wird der pH-Wert durch gleichzeitige Zugabe von 25 %iger $NH_3$-Lösung bei pH 8 gehalten. Der entstandene Niederschlag wird 12 h nachgerührt, dann abgesaugt und ausgewaschen. Er wird bei 60 °C/16 h getrocknet.

Borphosphate als Katalysatoren für das erfindungsgemäße Verfahren kann man beispielsweise durch Mischen und Kneten von konzentrierter Borsäure und Phosphorsäure und durch anschließende Trocknung und Calcination in Inertgas-, Luft- oder Dampfatmosphäre bei 250 bis 650 °C, vorzugsweise 300 bis 500 °C herstellen.

Geeignete saure Katalysatoren sind z.B. auch die sauer wirkenden Oxide von Elementen der III. und IV. Hauptgruppe sowie der IV. bis VI. Nebengruppe des periodischen Systems, insbesondere Oxide wie

Siliciumdioxid in Form von Kieselgel, Kieselgur, Quarz, weiterhin Titandioxid, Zirkondioxid, Vanadiumpentoxid, Nioboxid, Bortrioxid, Aluminiumoxid, Chromoxide, Molybdänoxide, Wolframoxide oder Gemische dieser Oxide.

Auch mit Phosphorsäure oder Borsäure getränkte Katalysatoren können eingesetzt werden. Phosphorsäure oder Borsäure wird auf $SiO_2$-, $Al_2O_3$- oder Bims-Träger, z.B. durch Auftränken oder Versprühen aufgebracht. Ein phosphorsäurehaltiger Katalysator kann beispielsweise durch Auftränken von $H_3PO_4$- oder $NaH_2PO_4$- oder $Na_2HPO_4$-Lösung auf $SiO_2$ und anschließende Trocknung bzw. Calcination erhalten werden. Phosphorsäure kann aber auch mit Kieselgel zusammen in einem Sprühturm versprüht werden; danach schließt sich eine Trocknung und meist eine Calcination an. Phosphorsäure kann auch in einer Imprägniermühle auf das Trägermaterial aufgesprüht werden.

Die hier beschriebenen Katalysatoren können wahlweise als 2- bis 4-mm-Stränge oder als Tabletten mit 3 bis 5 mm Durchmesser oder als Splitt mit Teilchengrößen von 0,1 bis 0,5 mm oder als Wirbelkontakt eingesetzt werden.

Die für die erfindungsgemäße Umwandlung der Oxy-, Alkoxy- oder Carboxiketone in der Regel gewählten Reaktionsbedingungen sind in der bevorzugten Gasphase 100 bis 500 °C, z.B. 150 bis 450 °C und insbesondere 300 bis 400 °C und eine Katalysatorbelastung WHSV von 0,1 bis 20 $h^{-1}$, insbesondere von 1,0 bis 10,0 $h^{-1}$ g Keton je g Katalysator und Stunde.

Die Gasphasenreaktion kann in einem Festbett oder in einem Wirbelbett ausgeführt werden.

Es ist auch möglich, die Reaktion in der Flüssigphase, in Suspension-, Riesel- oder Sumpffahrweise, bei Temperaturen zwischen 50 und 200 °C durchzuführen.

Das Verfahren wird im allgemeinen bei Normaldruck oder je nach Flüchtigkeit der Ausgangsverbindung bei vermindertem oder mäßig erhöhtem Druck diskontinuierlich, vorzugsweise kontinuierlich durchgeführt.

Schwerflüchtige oder feste Ausgangsstoffe kann man in gelöster Form, z.B. in THF-, Toluol- oder Petrolether-Lösung einsetzen. Es ist auch eine Verdünnung des Ausgangsstoffes mit derartigen Lösungsmitteln oder mit Inertgasen wie $N_2$, Ar, $H_2O$-Dampf möglich.

Nach der Umsetzung werden die entstandenen Produkte durch übliche Verfahren, z.B. durch Destillation aus dem Reaktionsgemisch isoliert; nichtumgesetzte Ausgangsstoffe werden gegebenenfalls in die Umsetzung zurückgeführt.

Beispiele 1 bis 19

Die Reaktion wird in der Gasphase unter isothermen Bedingungen in einem Rohrreaktor (Wendel, 0,6 cm Innendurchmesser, 90 cm Länge) mindestens 6 Stunden lang durchgeführt. Die Reaktionsprodukte werden durch übliche Methoden abgetrennt und charakterisiert. Die quantitative Bestimmung der Reaktionsprodukte und der Ausgangsstoffe erfolgt gaschromatographisch.

Für die Beispiele werden Katalysatoren eingesetzt.

Katalysator A

Ein Borosilikatzeolith des Pentasil-Typs wird in einer hydrothermalen Synthese aus 640 g hochdispersem $SiO_2$, 122 g $H_3BO_3$, 8000 g einer wäßrigen 1,6-Hexandiamin-Lösung (Mischung 50:50 Gew.%) bei 170 °C unter autogenem Druck in einem Rührautoklaven hergestellt. Nach Abfiltrieren und Auswaschen wird das kristalline Reaktionsprodukt bei 100 °C/24 h getrocknet und bei 500 °C/24 h calciniert. Dieser Borosilikatzoelith setzt sich zusammen aus 94,2 Gew.% $SiO_2$ und 2,3 Gew.% $B_2O_3$. Daraus werden durch Verformen mit einem Verformungshilfsmittel 2 mm-Stränge hergestellt, die bei 110 °C/16 h getrocknet und bei 500 °C/24 h calciniert werden.

Katalysator B

Ein Aluminosilikatzeolith vom Pentasil-Typ wird unter hydrothermalen Bedingungen bei autogenem Druck und 150 °C aus 65 g hochdispersem $SiO_2$, 20,3 g $Al_2(SO_4)_3$ x 18 $H_2O$ in 1 kg einer wäßrigen 1,6-Hexandiamin-Lösung (Mischung 50:50 Gew.%) in einem Rührautoklaven hergestellt. Nach Abfiltrieren und Auswaschen wird das kristalline Reaktionsprodukt bei 110 °C/24 h getrocknet und bei 500 °C/24 h calciniert. Der Aluminosilikatzeolith enthält 96,1 Gew.% $SiO_2$ und 4,6 Gew.% $Al_2O_3$. Der Katalysator wird zu 2 mm-Strängen verformt, bei 110 °C/16 h getrocknet und bei 500 °C/24 h calciniert.

Katalysator C

Ein Eisensilikatzeolith des Pentasil-Typs wird unter hydrothermalen Bedingungen bei autogenem Druck und 165 °C aus 273 g Wasserglas, gelöst in 253 g einer wäßrigen 1,6-Hexandiamin-Lösung (Mischung 50:50 Gew.%) und 31 g Eisensulfat, gelöst in 21 g 96 %iger Schwefelsäure und 425 g Wasser in einem Rührautoklaven während 4 Tagen synthetisiert. Der Zeolith wird abfiltriert, ausgewaschen, bei 110 °C/24 h getrocknet und bei 500 °C/24 h calciniert. Man erhält einen Eisensilikatzeolithen mit einem $SiO_2/Fe_2O_3$-Verhältnis von 17,7 und einem $Na_2O$-Gehalt von 1,2 Gew.%. Der Zeolith wird mit hochdispersem $SiO_2$ im Gewichtsverhältnis 80:20 zu 2,5 mm-Strängen verstrangt, bei 110 °C/16 h getrocknet und bei 500 °C/24 h calciniert.

Katalysator D

Ein handelsüblicher Na-Y-Zeolith wird mit wäßriger $(NH_4)_2SO_4$-Lösung in bekannter Weise ionenausgetauscht, so lange bis der Na-Gehalt kleiner 0,05 Gew.% ist (nach Trocknung 110 °C/2 h und Calcination bei 570 °C/3 h).

Das so erhaltene Pulver wird mit Verformungshilfsmitteln zu Strängen verformt, bei 110 °C getrocknet und bei 500 °C/16 h calciniert.

Katalysator E

Katalysator E erhält man, indem man Katalysator A mit $Ce(NO_3)_3$-Lösung imprägniert, danach bei 130 °C/2 h trocknet und bei 540 °C/2 h calciniert. Der Ce-Gehalt beträgt 1,7 Gew.%.

Katalysator F

Katalysator F wird wie Katalysator E hergestellt, jedoch mit einem Ce-Gehalt von 4,1 Gew.%.

Katalysator G

Katalysator G wird wie Katalysator E hergestellt, jedoch mit $Cs_2CO_3$- statt $Ce(NO_3)_3$-Lösung getränkt. Der Cs-Gehalt beträgt 0,7 Gew.%.

Katalysator H

Katalysator H erhält man, indem man den Borosilikatzeolith aus Katalysator A mit hochdispersem $SiO_2$ im Gewichtsverhältnis 70:30 zu 2 mm-Stängen verformt, bei 110 °C/16 h trocknet und bei 500 °C/16 h calciniert. Diese Stränge werden mit einer wäßrigen $NaH_2PO_4$ imprägniert, bei 110 °C getrocknet und bei 500 °C/14 h calciniert. Der Na-Gehalt beträgt 5 Gew.% und der P-Gehalt 7,5 Gew.%.

Katalysator I

$AlPO_4$-9 (APO-9) wird synthetisiert, indem man 200 g 98 %ige Phosphorsäure und 136 g Boehmit in 400 g Wasser löst bzw. suspendiert, hierzu eine wäßrige Lösung aus 112 g Diazabicyclo-2,2,2-octan (DABCO) und 320 g $H_2O$ zugibt und diese Mischung in einem Rührautoklaven bei 200 °C während 336 Stunden unter autogenem Druck umsetzt. Nach Abfiltrieren wird das kristalline Material bei 120 °C getrocknet und 500 °C/16 h calciniert. Das so synthetisierte $AlPO_4$-9 enthält 49,0 Gew.% $P_2O_5$, 37,1 Gew.% $Al_1O_3$. Dieses Material wird mit Verstrangungshilfsmitteln zu 3 mm-Strängen verformt, abermals bei 120 °C getrocknet und bei 500 °C/6 h calciniert.

Katalysator J

$SiAlPO_4$-5 (SAPO-5) wird aus einer Mischung aus 200 g 98 %ige Phosphorsäure, 136 g Boehmit, 60 g Kieselsol (30 %ig), 287 g Tripropylamin und 587 g $H_2O$ hergestellt. Diese Mischung wird bei 150 °C während 168 Std. unter autogenem Druck umgesetzt. Nach Filtration wird das kristalline Produkt bei 120 °C getrocknet und bei 500 °C calciniert. SAPO- 5 enthält 49,8 Gew.% $P_2O_5$, 33,0 Gew.% $Al_2O_3$, 6,2 Gew.% $SiO_2$. SAPO-5 wird mit einem Verstrangungsmittel zu 3 mm-Strängen verformt, bei 120 °C getrocknet und bei 500 °C calciniert.

Katalysator K

$CePO_4$ wird durch Fällung aus 52 g $Ce(NO_3)_3$ x 6 $H_2O$ und 56 g $NaH_2PO_4$ x 2 $H_2O$ erhalten. Nach der Filtration wird das Material zu Strängen verformt, bei 120 °C getrocknet und bei 450 °C calciniert. Katalysator K enthält 47,1 Gew.% Ce und 12,7 Gew.% P.

Katalysator L

Im Handel erhältliches $Zr_3(PO_4)_2$ wird mit Verformungshilfsmitteln zu 2 mm-Strängen verformt, bei 110 °C getrocknet und bei 500 °C/16 h calciniert.

Katalysator M

Nioboxidhydrat wird bei 300 °C/2 h calciniert und danach mit hochdispersem $SiO_2$ im Gewichtsverhältnis 70:30 zu 2 mm-Stängen verformt. Die Stränge werden bei 110 °C getrocknet und bei 300 °C/2 h calciniert.

Katalysator N

Im Handel erhältliches $TiO_2$ wird mit Verstrangungshilfsmitteln zu 2 mm-Strängen verformt, bei 110 °C getrocknet und bei 500 °C/16 h calciniert.

Katalysator O

D 10-10® (® = eingetragenes Warenzeichen) ($Al_2O_3$) wird mit $H_3BO_3$ gelöst in $CH_3OH$ getränkt, bei 120 °C getrocknet und bei 500 °C/15 h calciniert. Der Borgehalt beträgt 15 Gew.%.

In der nachfolgenden Tabelle 1 sind die mit den voranbeschriebenen Katalysatoren A bis O erzielten Ergebnisse sowie die Reaktionsbedingungen aufgeführt. Die Proben wurden nach 6 Stunden Reaktionszeit analysiert. Nach längerem Stehen bildet sich das Dimere des Isopropenylmethylketons. Dies kann durch Zugabe z.B. von Hydrochinon und durch Kühlung unterdrückt werden.

Tabelle 1 — 3-Hydroxi -3-methyl-butan-2-on ——> Isopropenylmethylketon

| Beispiel | 1 | 2 | 3 | 4 | 5 | 6 | 7 | 8 | 9 | 10 | 11 | 12 | 13 | 14 | 15 | 16 |
|---|---|---|---|---|---|---|---|---|---|---|---|---|---|---|---|---|
| Katalysator | A | A | E | F | G | H | B | C | D | I | J | K | L | M | N | O |
| Temperatur | 300°C | 400°C | 400°C | 400°C | 400°C | 400°C | 300°C | 400°C | 400°C | 400°C | 400°C | 400°C | 400°C | 400°C | 400°C | 400°C |
| WHSV | 2 $h^{-1}$ | 2 $h^{-1}$ | 2 $h^{-1}$ | 2 $h^{-1}$ | 2 $h^{-1}$ | 2 $h^{-1}$ | 2 $h^{-1}$ | 3 $h^{-1}$ | 2 $h^{-1}$ | 1 $h^{-1}$ | 2 $h^{-1}$ | 2 $h^{-1}$ | 2 $h^{-1}$ | 2 $h^{-1}$ | 2 $h^{-1}$ | 2 $h^{-1}$ |
| Umsatz % | 96,8 | 100 | 100 | 100 | 100 | 99,1 | 100 | 96,4 | 92,5 | 95,3 | 98,3 | 88,3 | 21,5 | 98,7 | 10,3 | 51,2 |
| Selektivität | | | | | | | | | | | | | | | | |
| I | 88,9 | 87,8 | 96,1 | 92,7 | 89,7 | 94,0 | 95,3 | 79,3 | 94,5 | 92,0 | 53,5 | 87,7 | 99,5 | 57,1 | 84,2 | 45,4 |
| II | 5,7 | 5,3 | 1,2 | 2,0 | 4,4 | 3,0 | 0,1 | 3,6 | - | 1,0 | 15,1 | 1,6 | - | 16,3 | - | - |

Aus den Beispielen Tabelle 1) erkennt man, daß unter den Katalysatoren für das erfindungsgemäße Verfahren Zeolithe besonders gut geeignet sind.

Beispiel 17

In einem Reaktor, der mit 100 g Katalysator A gefüllt ist, und der in einem Salzbad auf 400 °C thermostatisiert wird, werden 100 g/h eines Gemisches aus 3-Hydroxy-3-methyl-butan-2-on und THF (50 g : 50 g) 10 Tage lang vollständig umgesetzt. Nach dieser Laufzeit war noch keine Desaktivierung des

Katalysators feststellbar. Die während dieser Zeit erzielten Ergebnisse sind in Tabelle 2 zusammengestellt.

Tabelle 2

3-Hydroxy-3-methyl-butan-2-on $\xrightarrow{-H_2O}$ Methylisopropenylketon (I)

| Zeit | 20 h | 52 h | 88 h | 120 h | 152 h | 188 h | 213 h | 240 h |
|---|---|---|---|---|---|---|---|---|
| Fl.-% I | 89,1 | 90,9 | 92,2 | 93,2 | 93,5 | 94,3 | 94,8 | 94,5 |

Beispiel 18

3-Hydroxy-3-methylpentan-2-on wird mit THF 50 g : 50 g gemischt und bei 400 °C mit WHSV = 1,9 $h^{-1}$ am Katalysator A vollständig umgesetzt. Es werden im flüssigen Austrag 75,5 Fl.-% 3-Methyl-pent-3-en-2-on und dessen Isomere erhalten.

Beispiel 19

3-Pentamethylen-3-hydroxypropan-2-on wird mit THF 50 g : 50 g gemischt und bei 400 °C mit WHSV = 4,3 $h^{-1}$ am Katalysator B vollständig umgesetzt. Es werden im flüssigen Austrag 67,4 Fl.-% Cyclohexenylmethylketon und dessen Isomere erhalten.

## Patentansprüche

**1.** Verfahren zur Herstellung von alpha,beta-ungesättigten Ketonen der Formel I

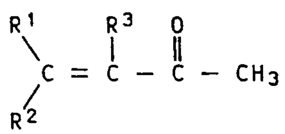

(I),

in der $R^1$, $R^2$, $R^3$ Wasserstoff, Alkyl-, Alkenyl- mit 1 bis 12 Kohlenstoffatomen, Cycloalkyl-, Aryl-, Aralkyl- bzw. Alkylarylreste, die ihrerseits substituiert sein können, bedeuten, bzw. $R^1$ und $R^3$ oder $R^2$ und $R^3$ zusammen mit den C-Atomen, an die sie gebunden sind, ein Cycloalkan oder Cycloalken bilden können, dadurch gekennzeichnet, daß man Ketone der Formel (II)

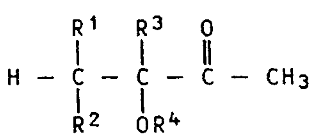

(II),

in der $R^1$, $R^2$, $R^3$ obige Bedeutung haben und $R^4$ einen Wasserstoff, Alkyl- oder Carboxylrest bedeutet, in Gegenwart von sauren Heterogenkatalysatoren umsetzt.

**2.** Verfahren nach Anspruch 1, dadurch gekennzeichnet, daß man als Heterogenkatalysatoren Zeolithe verwendet.

**3.** Verfahren nach den Ansprüchen 1 und 2, dadurch gekennzeichnet, daß man als Heterogenkatalysatoren Zeolithe des Pentasiltyps verwendet.

**4.** Verfahren nach den Ansprüchen 1 und 2, dadurch gekennzeichnet, daß man als Heterogenkatalysatoren Aluminiumsilikatzeolithe des Faujasittyps verwent.

5. Verfahren nach den Ansprüchen 1 bis 4, dadurch gekennzeichnet, daß man Heterogenkatalysatoren verwendet, die mit Alkalimetallen, Erdalkalimetallen, Übergangsmetallen oder mit Seltenen Erden dotiert sind.

6. Verfahren nach Anspruch 1, dadurch gekennzeichnet, daß man als Heterogenkatalysatoren Phosphate verwendet.

7. Verfahren nach den Ansprüchen 1 und 6, dadurch gekennzeichnet, daß man als Heterogenkatalysatoren hydrothermal hergestellte Aluminiumphosphate oder Siliciumaluminiumphosphate oder Siliciumeisenaluminiumphosphate oder Eisenaluminiumphosphate verwendet.

8. Verfahren nach Anspruch 1, dadurch gekennzeichnet, daß man als Heterogenkatalysatoren Metalloxide des Al, B, Ti, Zr, Si oder deren Gemische verwendet.

9. Verfahren nach Anspruch 1, dadurch gekennzeichnet, daß man als Heterogenkatalysatoren Phosphorsäure auf Bims bzw. Siliciumdioxid bzw. Aluminiumoxid oder Borsäure auf Bims bzw. Siliciumdioxid bzw. Aluminiumoxid verwendet.

## Claims

1. A process for the preparation of an alpha,beta-unsaturated ketone of the formula I

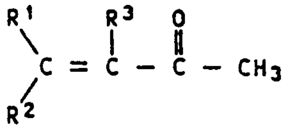

I

where $R^1$, $R^2$ and $R^3$ are each hydrogen, alkyl, alkenyl of 1 to 12 carbon atoms, cycloalkyl, aryl, aralkyl or alkylaryl, which in turn may be substituted, or $R^1$ and $R^3$ or $R^2$ and $R^3$ together with the carbon atoms to which they are bonded may form a cycloalkane or cycloalkene radical, wherein a ketone of the formula II

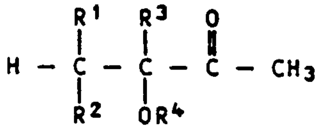

II

where $R^1$, $R^2$ and $R^3$ have the above meanings and $R^4$ is hydrogen, alkyl or carboxyl, is converted in the presence of an acidic heterogeneous catalyst.

2. A process as claimed in claim 1, wherein the heterogeneous catalyst used is a zeolite.

3. A process as claimed in claim 1 or 2, wherein the heterogeneous catalyst used is a zeolite of the pentasil type.

4. A process as claimed in claim 1 or 2, wherein the heterogeneous catalyst used is an aluminosilicate zeolite of the faujasite type.

5. A process as claimed in any of claims 1 to 4, wherein a heterogeneous catalyst which is doped with an alkali metal, an alkaline earth metal, a transition metal or a rare earth metal is used.

6. A process as claimed in claim 1, wherein the heterogeneous catalyst used is a phosphate.

7. A process as claimed in claims 1 and 6, wherein the heterogeneous catalyst used is a hydrothermally prepared aluminum phosphate or silicon aluminum phosphate or silicon iron aluminum phosphate or iron aluminum phosphate.

8. A process as claimed in claim 1, wherein the heterogeneous catalyst used is an oxide of Al, B, Ti, Zr or Si or a mixture of these.

9. A process as claimed in claim 1, wherein the heterogeneous catalyst used is a phosphoric acid on pumice or silica or alumina, or boric acid on pumice or silica or alumina.

**Revendications**

1. Procédé de préparation de cétones alpha-bêta-insaturées de la formule I

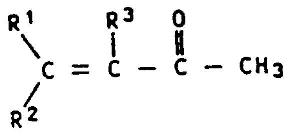

(I),

dans laquelle $R^1$, $R^2$ et $R^3$ représentent des atomes d'hydrogène, des radicaux alkyle, alcényle, qui comportent de 1 à 12 atomes de carbone, des radicaux cycloalkyle, aryle, aralkyle ou alkylaryle, qui peuvent eux-mêmes être substitués, ou bien $R^1$ et $R^3$ ou $R^2$ et $R^3$ peuvent former, ensemble avec les atomes de carbone auxquels ils sont liés, un cycloalcane ou cycloalcène, caractérisé en ce que l'on fait réagir des cétones de la formule (II)

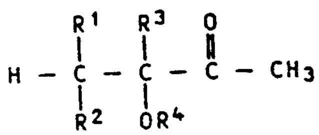

(II),

dans laquelle $R^1$, $R^2$ et $R^3$ possèdent les significations qui leur ont été attribuées ci-dessus et $R^4$ représente un atome d'hydrogène, un radical alkyle ou carboxyle, en présence de catalyseurs hétérogènes acides.

2. Procédé suivant la revendication 1, caractérisé en ce que l'on utilise des zéolites à titre de catalyseurs hétérogènes.

3. Procédé suivant les revendications 1 et 2, caractérisé en ce que l'on utilise des zéolites du type pentasile à titre de catalyseurs hétérogènes.

4. Procédé suivant l'une quelconque des revendications 1 et 2, caractérisé en ce que l'on utilise des zéolites de silicate d'aluminium du type faujasite à titre de catalyseurs hétérogènes.

5. Procédé suivant l'une quelconque des revendications 1 à 4, caractérisé en ce que l'on utilise des catalyseurs hétérogènes qui sont dopés avec des métaux alcalins, des métaux alcalino-terreux, des métaux de transition ou des terres rares.

6. Procédé suivant la revendication 1, caractérisé en ce que l'on utilise des phosphates à titre de catalyseurs hétérogènes.

7. Procédé suivant l'une quelconque des revendications 1 et 6, caractérisé en ce que l'on utilise, à titre de catalyseurs hétérogènes, des phosphates de fer et d'aluminium, des phosphates de silicium, de fer et d'aluminium, des phosphates de silicium et d'aluminium, ou des phosphates d'aluminium, préparés par voie hydrothermique.

8. Procédé suivant la revendication 1, caractérisé en ce que l'on utilise des oxydes des émtaux Al, B, Ti, Zr, Si, ou leurs mélanges, à titre de catalyseurs hétérogènes.

9. Procédé suivvant la revendication 1, caractérisé en ce que l'on utilise, à titre de catalyseurs hétérogènes, de l'acide phosphorique sur pierre ponce ou du dioxyde de silicium ou de l'oxyde d'aluminium, ou

bien de l'acide borique sur de la pierre ponce ou du dioxyde de silicum ou de l'oxyde d'aluminium.